# EUROPÄISCHE PATENTSCHRIFT

(11) **EP 4 405 039 B1**
(45) Veröffentlichungstag und Bekanntmachung des Hinweises auf die Patenterteilung: **04.11.2026**
(21) Anmeldenummer: 22783042.9
(22) Anmeldetag: 26.09.2022
(51) Int. Cl.: A61N 5/10

(54) **VERFAHREN UND TECHNISCH FUNKTIONALE GUI ZUR AUFFINDEN EINES THERAPIEPLANS UNTER PLANVORSCHLÄGEN ZUR KREBSTHERAPIE MIT INTERAKTIVER FILTERUNG (VERFAHREN, TECHNISCH FUNKTIONALE GUI)**
METHOD AND TECHNICALLY FUNCTIONAL GUI FOR FINDING A TREATMENT PLAN AMONG RECOMMENDED PLANS FOR TREATING CANCER, USING AN INTERACTIVE FILTER
PROCÉDÉ ET GUI TECHNIQUEMENT FONCTIONNELLE DE RECHERCHE D'UN PLAN DE TRAITEMENT PARMI DES PROPOSITIONS DE PLANS POUR LE TRAITEMENT DU CANCER FAISANT INTERVENIR UN FILTRAGE INTERACTIF

(30) Priorität: 25.09.2021 DE 102021124813
(43) Veröffentlichungstag der Anmeldung: 31.07.2024
(73) Patentinhaber: Fraunhofer-Gesellschaft zur Förderung der angewandten Forschung e.V., 80686 München (DE)
(72) Erfinder: KUEFER, Karl-Heinz, 67685 Weilerbach (DE); SUESS, Philipp, 55257 Budenheim (DE); COLLICOTT, Cristina, 79206 Breisach (DE)
(74) Vertreter: Leonhard, Frank Reimund
(86) Internationale Anmeldenummer: PCT/IB2022/059067
(87) Internationale Veröffentlichungsnummer: WO 2023/047369

(56) Entgegenhaltungen:
- US-A1- 2011 085 643
- US-B2- 10 512 790
- MONZ M ET AL: "Pareto navigation-algorithmic foundation of interactive multi-criteria IMRT planning; Pareto navigation", PHYSICS IN MEDICINE AND BIOLOGY, INSTITUTE OF PHYSICS PUBLISHING, BRISTOL GB, vol. 53, no. 4, 21 February 2008 (2008-02-21), pages 985 - 998, XP020127372, ISSN: 0031-9155

## Beschreibung

Diese Offenbarung (und Ansprüche) betreffen ein Verfahren der multikriteriellen Optimierung (MCO). Auch betroffen ist eine GUI, eigenständig, oder zur Anwendung (Verwendung) in diesem Verfahren.

Die Erfindung befasst sich mit einem Verfahren zum Optimieren eines eingestellten Zustands einer Maschine für eine Tumorbehandlung (RT Maschine, Strahlengerät oder therapeutisches Gerät). Insbesondere geht es dabei um eine erhebliche Verbesserung eines Systems nach US 7,391,026 B2. Die dort offenbarte Steuerung einer Planungsfindung zur Festlegung eines bestmöglichen Plans zur Behandlung eines Patienten, der unter einer Tumorerkrankung leidet, war ein Quantensprung. Es war eine Umkehr der bisher vorgeschlagenen Vorgehensweisen und ist für einen speziellen Fall der Strahlentherapie unter IMRT im Fachgebiet bekannt. Die "inverse Therapieplanung" genannte Vorgehensweise wurde von Bortfeld vorgeschlagen, vgl. US 7,391,026 B2, Spalte 1, Zeile 43 ff. Im Laufe der Zeit, unter Anwendung sowie praktischer Erprobung haben sich Verbesserungsmöglichkeiten eröffnet, bei der Navigation, bei der Entscheidungsunterstützung und der Genauigkeit der gefundenen Lösung als Therapieplan. Auch von Interesse ist die Vereinfachung der mathematischen Berechnung der zu findenden Lösung oder Lösungen.

Eine Lösung im Sinne dieser Offenbarung ist ein ausgewählter Plan, der als Therapieplan eingestellt oder vorgegeben werden soll. Dabei hat der Benutzer oder Planer (kurz User) eine Vielzahl von Planvorschlägen, die auch alle Lösungen darstellen, die aber noch Optimierungsbedarf haben oder sagen wir, Verbesserungsmöglichkeiten eröffnen.

Geht man vom genannten Stand der Technik aus, findet der User anhand eines Entwurfswerkzeugs einen geeigneten Plan. Der geeignete Plan ist weitreichend und er enthält Einstellparameter zur Einstellung eines (oder: am) therapeutischen Gerät, welches die Tumortherapie später an einem Patienten ausführt. Entsprechend deutlich ist, dass die Einstellung dieses Geräts und die Erstellung oder Ermittlung der Einstellung des Geräts noch keine therapeutische Behandlung und auch keine medizinische Behandlung als solche ist, sondern eine davor liegende Vorstufe.

Gefunden werden Einstellparameter technischer Natur, mit denen eine Strahlentherapie später und ggf. an völlig anderer Stelle durchgeführt werden kann. Diese nennt die genannte Strahlentherapie einen Plan. Eine IMRT ist hierbei eine besondere Form der Strahlentherapie. Letzterer Begriff ist die allgemeine Behandlung. Der Plan ist gleichzeitig auch "eine Lösung", ausgewählt aus einer Vielzahl von vorberechneten Plänen oder Lösungen, die alle geeignet sind, aber nur eine davon ist die für den User Bestmögliche.

Diese eine Bestmögliche aus der Vielzahl schon zur Verfügungen stehenden Plänen (sprich Planvorschlägen) auszuwählen, ist gemäß US 7,391,026 B2 möglich.

Mit WO 2012 069999 A1 wurde der ursprüngliche Plan (Therapieplan oder Behandlungsplan) nur lokal verändert, nicht aber der ganze Plan. Der Fachmann würde dabei davon sprechen, dass unter "praktischer Beibehaltung des Ausgangsplans" eine lokale Stelle, die als klein benannt werden kann, verändert wird. Würde er einen neuen Plan verwenden, würde er die lokalisierten kritischen Bereiche (die Critical Spots) zwar verändern, aber auch verlagern und im Allgemeinen nicht wirklich entfernen. Eine zu globale Veränderung des Plans wurde also vermieden. Anders gefasst, Änderungen am "ersten Plan" sollen außerhalb des/der "critical spots" so wenig als möglich erfolgen.

WO 2013 093852 A1 stellte dem Benutzer eine weitergehende, verfeinerte Variante an die Hand, die sich ein Stück von der Abstraktion der US 7,391,026 B2 löste, die für die Navigation bereitstand. Der Benutzer oder Planer sollte es an die Hand bekommen, in seinen geübten und herkömmlichen Bahnen auch denken zu können und eine wichtige Orientierung in diesen geübten, herkömmlichen Bahnen bieten DVH Kurvenscharen, welche dem Benutzer/Planer/Arzt (User) wesentliche Informationen für die Qualität eines 'Plans' liefern. In der zuvor genannten Schrift hatte diese DVH-Kurvenschar nur eine darstellende Funktion, sie war (dort in Figur 6, links unten) eine Folge eines anderweitig festgelegten 'Plans'.

Das Darstellungsverfahren der WO 2013 093852 A1 arbeitet mit DVH-Kurvenscharen und diese werden visuell so dargestellt, dass ein Benutzer es in der (und an der) Hand hat, mit dieser Darstellung seine Planvorschläge zu entwickeln, respektive interaktiv Lösungen aus der gespeicherten Vielfalt von Lösungen (entspricht den Planvorschlägen) herauszufinden. Das Verfahren stellt eine Vielzahl von vorberechneten Lösungen optisch sichtbar dar. Dies erfolgt auf einer Darstellungseinrichtung, die einen gebräuchlichen Sichtschirm beinhaltet. Die Vielzahl von vorberechneten Lösungen für mögliche Pläne, die vorberechnet in einer Datenbank gespeichert vorliegen, wird auf der Darstellungseinrichtung so präsentiert, dass es dem Benutzer ermöglicht wird, mit dieser Lösungsvielfalt zu arbeiten. Ein Startpunkt wird dazu auf einer der DVH Kurven ausgewählt. Dieser Startpunkt liegt auf der ausgewählten DVH Kurve, von denen mehrere im Hauptdiagramm dargestellt sind. Diese als "Kurvenschar" benannte Darstellung repräsentiert eine Lösung der Datenbank. Auf einer Kurve dieser Kurvenschar ist oder wird der Punkt als Startpunkt ausgewählt. Das System ordnet diesem Punkt eine gerade Achse zu, die durch den Startpunkt verläuft. Die gerade Achse schneidet die ausgewählte Kurve am Startpunkt oder andersherum, der Startpunkt definiert sowohl die Lage der Achse, wie auch die ausgewählte Kurve. Um den Startpunkt herum wird ein Steuerbereich (als Navigationsabschnitt oder -fenster) deutlich gemacht, den der Benutzer "als optisch hervorgehoben" wahrnimmt. Mit der "wirkungsmäßigen optischen Hervorhebung" dieses Steuerbereichs (Abschnitts) wird dem Benutzer die Vielfalt von in der Datenbank gespeicherten Lösungen visualisiert. Er erhält eine Möglichkeit, diese Vielfalt in ihrer Darstellung zu steuern und für die Darstellung auszuwählen. Die Vielzahl von derzeit nicht dargestellten Lösungen (alle Lösungen, die gespeichert sind, abzüglich der einen, im Hauptdiagramm dargestellten Lösung) kann so visualisiert werden. Sie wird noch nicht dargestellt, da nur eine der Vielzahl von Lösungen im Hauptdiagramm darstellt ist, aber sie wird angedeutet durch den Steuerbereich, der um den genannten Startpunkt herum liegt und - optisch wirkend - hervorgehoben ist.

WO 2005 035061 A2 - Riker, Nomos nahm eine IMRT Optimierung in einer DVH Umgebung vor, dort Abs. 131 und 117. Die folgende Kurzbeschreibung bezieht die Bezugszeichen der dortigen Figur 4 ein. Die Optimierung arbeitet mit Schiebereglern und hat eine Rückgängig Funktion (partial undo slider, dort 155). Der partielle Rückgängig-Schieber 155 entspricht im Grunde einer Rücktaste, die automatisch die letzte vom Benutzer vorgenommene Einstellung übernimmt (Bezugszeichen von dort übernommen). Außerdem befindet sich der Schieber 155 standardmäßig mit seinem Griff 157 in der äußersten rechten Position, wenn die betreffende Isodosenkontur 162 losgelassen wird und das entsprechende Scanfenster 160 den geänderten Plan anzeigt. Wenn der Benutzer den Slider 157 ganz nach links schiebt, macht der Algorithmus die Änderung vollständig rückgängig. Wenn der Benutzer den Schieber 157 wieder nach rechts schiebt, wird die Änderung vollständig wiederhergestellt, so dass der Benutzer die Auswirkungen der Änderung in Echtzeit erkennen kann, und zwar vollständig oder schrittweise, während er den Griff des Sliders hin und her schiebt. Auf diese Weise kann der Benutzer das Ausmaß der Kompromisse besser nachvollziehen. Der Benutzer kann einen beliebigen Zwischenpunkt auswählen, um die Plankonfiguration vor der Anpassung und das Ergebnis der Anpassung zu sehen.

Die Grundlagen die, wie in einem Navigations-Raum unter Anwendung der Pareto-Grundsätze navigiert wird, ergibt sich aus Monz et al 2008 Phys. Med. Biol. 53 985 (algorithmic foundation of interactive multi-criteria IMRT planning), Autoren Monz, Küfer, Bortfeld and Thieke. Die Pareto-Navigation ist dabei ein interaktives multikriterielles Optimierungsverfahren welche von den Navigationsmechanismen "Auswahl" und "Einschränkung" getragen wird. Erstere Auswahl erlaubt die Formulierung von Wünschen, während die letztere Einschränkung den Ausschluss unerwünschter Pläne ermöglicht. Sie werden als Optimierungsprobleme auf dem sogenannten Planbündel realisiert - einer Menge die aus vorberechneten Plänen besteht (auch hier Planvorschläge). Sie können näherungsweise so umformuliert werden, dass ihre Lösungszeit unter einem Bruchteil einer Sekunde beträgt. Dem User wird unmittelbar eine Rückmeldung über seine Entscheidungen gegeben. Die Pareto-Navigation ermöglicht eine Manipulation des aktuellen Plans und der Menge der betrachteten Pläne in Echtzeit. Änderungen werden ausgelöst durch einfache Mausoperationen, z.B. auf einem sogenannten Navigationsstern oder einer Gruppe von auf einem Display dargestellten Bedienhilfen, im Beispiel als Slider, die jeweils ein Kriterium oder eine Zielgröße verändern - und führen zur Aktualisierung des Navigationssterns oder der anderen Slider in Echtzeit (korrekt, des Handles des Sliders). Dosisvisualisierungen ändern sich so in der Planung mit interaktiver Rückkopplung zum User. Einer der gefundenen Pläne kann dann oder wird dann ausgewählt, um ihn auf Therapiegeräten vorzugeben oder einzustellen. Erst später, also nach der Einstellung, kann ein Patient mit dieser Einstellung auch behandelt werden.

Da jeder Pareto-optimale Plan im Planbündel mit wenigen Navigationsoperationen gefunden werden kann, ermöglicht ein zugehöriger Software Navigator eine schnelle und gezielte Planfindung. Damit erlaubt das Konzept eine multikriterielle Optimierung in Echtzeit und interaktive mit dem User. Die Pareto-Navigation bietet bisher unerreichte Echtzeit-Interaktionen, Benutzerfreundlichkeit und Planvielfalt.

**Ein technisches Problem** der hier offenbarten Erfindung liegt darin, die Einstellung von technischen Größen von durch Pareto Navigation gefundenen Therapieplänen verbessert zu ermöglichen, insbesondere die Entscheidungsfindung des Planers (auch User genannt) zu erleichtern und/oder die mathematische Berechnung der Lösung zu vereinfachen Anspruch 1 oder 22 schaffen eine Lösung.

Mit der beanspruchten Erfindung wird in einem ersten Aspekt dem Benutzer ein neuer Horizont eröffnet. Der bisherige Planer, der weiterhin User genannt werden soll, hat zwei verschiedene Arten von Plänen und Entscheidungskriterien dem abschließenden Planer, der Arzt genannt werden soll, vorbereitet. Die normale Navigation im Paretoraum ermöglicht das Navigieren auf Zielen, die aus der Dosisverteilung anhand einer DVH stammen können. Diese Ziele sind physikalische Werte oder Bewertungen, die auf dem Dosismittelwert eines Organs, oder allgemeiner einem Volume of Interest entsprechen. Anders als der Planer wird der Arzt aus seiner Gewohnheit an diesen Zielen nicht alleine interessiert sein, primär wird er eher aus der Fachliteratur oder aus klinischen Studien publizierte klinische Ziele bevorzugen, die andererseits in der Navigation und mit der Berechnung von Paretolösungen nicht oder nicht einfach umzusetzen sind.

Solche klinischen Ziele können dadurch vorgegeben werden, dass der den Plan abschließend festlegende Arzt Kriterien berücksichtigt wissen möchte, beispielsweise ein bestimmter Prozentsatz eines Organs soll eine Mindestdosis an Strahlung erhalten. Ein anderes klinisches Ziel ist die Vorgabe, dass zumindest eines von zwei vorhandenen Organen die Strahlentherapie überstehen soll. Ein weiteres klinisches Ziel ist es, dass die Behandlung so früh als möglich beginnen soll. Noch ein weiteres dieser klinischen Ziele ist, dass zum Beispiel 99 % eines Tumorvolumens mit einem Mindestwert an Strahlendosis versehen werden soll. Besonders logische Verknüpfungen von Zielen aus dem Navigationsraum, wie die oben genannte Forderung der Schonung mindestens eines von mehreren Organen desselben Patienten, lassen sich dort nicht mit den Werkzeugen einer Navigation erfassen oder umsetzen.

Der Arzt spricht bei der Festlegung eines Therapieplans oft eine andere Sprache, als der auf physikalische Größen bei der Navigation fokussierte User oder Planer. Natürlich kann auch der Arzt mit diesen physikalischen Werten umgehen, er wird sie indes nicht primär als Ziel annehmen oder abfragen. Unter den Zielen, die von ihm als klinische Ziele bevorzugt werden sind die oben genannten Beispiele. Diese sind mathematisch gesehen 'nichtkonvexe' Funktionen der physikalischen Dosis und es ist schwierig, in vorgegebener Zeit nichtkonvexe Funktionen zur Optimalität zu lösen.

Die Schaffung eines Entscheidungsraums, in dem keine Navigation im gewohnten Sinne stattfinden kann, bringt die Lösung. Hier kann der Arzt seine klinischen Ziele berücksichtigt finden. Die Berechnung der o.g. klinischen Ziele erfolgt aus den Dosis-Volumen-Constraints, also u.a. den Dosismittelwerten pro Organ (oder allgemeiner dem Volume of Interest). Aus diesen Dosiswerten, beispielsweise der Vorgabe einer Strahlendosis >70Gy für ein (gesamtes) physikalisches Ziel, wird ein klinisches Ziel errechnet, welches die Dosis für beispielsweise 99% des genannten Volumens auswertet und damit ein klinisches Ziel errechnet.

Mit der Erfindung wird eine erste große Menge von Punkten, die nicht nur pareto sein müssen, aus der Navigation erhalten, die im Anschluss in das klinische Ziele übersetzt oder umgerechnet wird. Je mehr Punkte es gibt, desto höher die Anzahl von Punkten im Entscheidungsraum. Das ist hier sinnvoll und an sich auch erforderlich, um ausreichend Punkte für die klinischen Ziele zu haben. Ausgesondert werden können solche, die fastpareto sind. Es ist dabei keine 1:1 Beziehung zwischen den Punkten des Navigationsraums und den Punkten im Entscheidungsraum, wo die klinischen Ziele aufgetragen werden. Die Dosismittelwerte mögen zwar nahe bei den klinischen Zielen liegen, dennoch ist eine unmittelbare Übertragung oder Abbildung nicht möglich. Und es ist ebenso unmöglich, aus den klinischen Zielen zu rekombinieren, also auf die physikalischen Bewertungen der Dosismittelwerte zurück zu rechnen.

Der Arzt findet den für ihn geeigneten Therapieplan unter der Vielzahl von Planvorschlägen nicht durch Navigation, sondern durch Verwendung von Restriktoren im Entscheidungsraum, mit denen er Planvorschläge abwählt, um zu seinem Wunschplan oder Zielplan zu gelangen. Je größer die erste Punktemenge ist, die aus dem Navigationsraum in den Entscheidungsraum übersetzt oder umgerechnet wird, desto genauer kann seine Auswahl werden. Die Navigation im Navigationsraum bietet dafür die Möglichkeit, da der User hier durch konvex kombinieren genügend Lösungen erstellt, die gespeichert werden können, und in den Entscheidungsraum umgerechnet werden.

Bei der Navigation wird Interpolation verwendet, sodass neue Punkte aus den schon vorberechneten Punkten entstehen. Die Umrechnung aller Punkte für den Entscheidungsraum kann diejenigen nach der Umrechnung entfallen lassen, die keine Pareto-Eigenschaft haben. Soll die Punktemenge, von der ausgegangen wird weiter vergrößert werden, können auch solche Punkte benutzt werden, welche keine Pareto-Eigenschaft haben. Es ist dabei keineswegs sichergestellt, dass Pareto Punkte auf der Eingangsseite auch zu Pareto Punkten auf der Ausgangsseite, also dem Entscheidungsraum führen.

Der im Anspruch erwähnte Toleranzbereich, der sich um das Paretooptimium bilden kann, ist veranlasst durch Rechenungenauigkeit, Lageungenauigkeit, Modellfehler oder numerische Ungenauigkeit.

Im Entscheidungsraum können auch solche Zielachsen aufgetragen werden, die für einen Volume of Interest einen Dosismittelwert angeben (allgemein: Eine mathematische (konvexe) Norm der Dosis).

Besonders logische Verknüpfungen von Zielen können nicht konvex kombiniert berechnet werden, eignen sich also nicht für den Navigationsraum. Beispiele für solche Ziele kann sein: Eine der Speicheldrüsen soll die Strahlentherapie überstehen, oder einer der beiden Sehapparate soll die Strahlentherapie überstehen, oder eines von einem doppelt vorhandenen Organ soll die Strahlentherapie überstehen.

Auch ein passives Ziel ist nicht für den Navigationsraum geeignet, bspw. die Wartezeit auf die Behandlung.

Die erste Punktemenge mit den physikalischen Bewertungen oder Werten von Dosiswerten kann größer als 1.000 sein. Das wird als Untergrenze einer "großen Punktemenge" angesehen.

Im Entscheidungsraum findet, wie oben gesagt, keine Navigation statt, unter der gemeinhin eine Selektion und eine Restriktion verstanden wird. Im Entscheidungsraum wird der Arzt auf Restriktionen beschränkt, hat aber aufgrund der Vielzahl von vorhandenen Planvorschlägen als Punkte eine hinreichende Auswahl. Er kann dazu hin ganze Technologien anwählen oder abwählen, und mehrere Zielachsen im Entscheidungsraum können zum Beispiel nach Priorität gruppiert werden.

Die Filterung im Entscheidungsraum durch Abwahl von nicht gewollten Planvorschlägen geschieht durch Restriktoren, die einen ganzen zusammenhängenden Bereich von Planvorschlägen abzuwählen in der Lage sind. Dazu wird ein Restriktor auf einem Slider gesetzt, um zwischen sich und dem Ende des Sliders alle dazwischenliegenden Punkte abzuwählen. Die Abwahl auf einem Slider bedingt die Abwahl des gesamten Planvorschlags, mit seinen Punkten auch auf den anderen Slidern.

Eine Scoring Funktion ermöglicht es dem Arzt für jede von mehreren verfügbaren Technologien eine Lösung als Planvorschlag angezeigt zu erhalten. Dieser Vorschlag erfolgt automatisiert und wird durch einen Restriktor der Vorschlag abgewählt, entsteht automatisiert ein weiterer Vorschlag aus den verfügbaren Planvorschlägen im Entscheidungsraum. Angezeigt werden kann der bestmögliche Vorschlag, oder der nächste Vorschlag, der aufgrund der verfügbaren Lösungsmenge in der Nähe ist.

Ein "Dosismittelwert" (im obigen Verständnis) kann je nach Organ unterschiedlich berechnet werden. Es gibt serielle Organe, beispielsweise das Rückenmark, oder parallele Organe, beispielsweise die Lunge, bei denen die Dosis Mittelwerte unterschiedlich berechnet werden.

Vorgeschlagen wird nach einem anderen Aspekt mit Anspruch 1 ein Verfahren zur Findung eines "guten" Plans unter vielen Planvorschlägen (als mögliche Lösungen) zur strahlenbasierten Krebstherapie, wobei jede Lösung einen Punkt in einem Navigationsraum darstellt, und eine Paretolösung bildet, die eine Vielzahl von Einstellparametern enthält, also festlegt, und in einem Maschinenraum die Einstellungen für abzugebende Strahlendosen und den Verlauf einer Strahlentherapie der Krebstherapie an der Therapiemaschine festlegt.

Für die beanspruchte Lösung ist es nicht vorgesehen, dass diese eingestellte oder vorgegebene Therapie auch durchgeführt wird. Die Lösung, welche hier beansprucht ist, ist bereits dann fertig, wenn die Einstellungen am Therapiegerät (der Therapiemaschine) erfolgt oder erfolgen kann. Eine spätere oder mit der Einstellung erfolgende Therapie ist nicht Gegenstand der Ansprüche. Der hier beschriebenen Erfindung geht es um die Findung eines bestmöglichen Plans für einen bestimmten Patienten, und allenfalls die Vorgabe der Einstell-Parameter an der Therapiemaschine.

Der Navigationsraum enthält zumindest eine Paretofront, hinter welcher verfügbare Behandlungspläne (Planvorschläge) liegen und unter denen von einem Benutzer navigiert wird. Alle verfügbaren Lösungen werden in einem Speicher vorgehalten und sind vorab berechnet. Sie werden zur Darstellung aus dem Speicher abgerufen oder ausgelesen. Das kann eine Datenbank sein. Die abgerufenen oder ausgelesenen Lösungen werden auf einem Display dargestellt. Die so verfügbaren Lösungen werden von dem Benutzer für seine Navigation benutzt. Für die Navigation können die Punkte der Planvorschläge nach ihren Zielen auf Zielachsen abgebildet werden, wobei die Navigation nach Art von Slidern erfolgen kann.

Es ist ein weiterer, separat dargestellter Entscheidungsraum vorgesehen, in dem die navigierbaren Punkte aus dem Navigationsraum auf Zielachsen abgebildet werden, ohne Möglichkeit, im Entscheidungsraum zu navigieren, indes nach Art eines Restriktors im Entscheidungsraum binär auszuwählen, welche Lösungen als Therapiepläne nicht benutzt werden.

Im Entscheidungsraum sind zumindest (eine oder mehrere) solche Zielachsen enthalten, die nicht im Navigationsraum zur Navigation dargestellt werden. Zielachsen, die sich nicht für den Navigationsraum eignen, sind solche, bei denen mathematisch nicht garantiert werden kann, dass in vorgegebener Zeit eine bestmögliche Lösung zu berechnen ist. Diese werden in den Entscheidungsraum ausgelagert.

Dort finden diese Zielachsen zur Benutzung durch den Planer/User ihren Platz. Ihre Benutzung auch im Navigationsraum würde die Berechnung von vordefinierten Paretolösungen erschweren, zumindest indes zu einem unerwarteten Verhalten für den Nutzer (bei der Navigation) führen, da die Slider sich nicht so verhalten würden wie der Planer/User sie bewegt.

Das bedeutet im Umkehrschluss indes nicht, dass keine Zielachsen im Entscheidungsraum dargestellt werden, die auch im Navigationsraum zur Navigation dargestellt werden. Also solche Zielachsen, die im Navigationsraum tatsächlich zur Navigation dargestellt werden. Solche Zielachsen, die nur zur Umgehung des Wortlauts das Anspruchs im Navigationsraum vorgesehen werden, sind wie Zielachsen zu werten, die dort nicht dargestellt werden.

Mit Zielachsen können die auf den Zielachsen abgebildeten Kriterien (auch: Ziele) Berücksichtigung finden, und zwar bei der Restriktion, mit welcher der User solche Pläne ausschließt. Im Entscheidungsraum soll das als eine Filterung betrachtet werden, es ist keine Navigation. Die Navigation selber, genauer, die Pareto-Navigation findet im Navigationsraum statt, wo quasi-stetige Veränderungen an den Plänen möglich sind, eine Interpolation zwischen benachbarten Punkten berechenbar ist und der User die primär Strahleneinfluss habenden Kriterien (als Ziele) auf Zielachsen berücksichtigen kann und sie zur Navigation heranziehen wird. Der Begriff der quasi-stetige Veränderungen ist mit Blick auf die Rasterung oder Diskretisierung der Planvorschläge durch die Pareto-Navigation und die für sie mögliche Interpolation zwischen benachbarten Punkten nahezu kontinuierlich.

Unter Beispielen, die sich nicht für eine Navigation eignen, sind folgende "gröberen Ziele" zu finden. Die Zeit bis zu einer möglichen Behandlung auf einem oder jedem der mehreren verfügbaren Therapiemaschinen (Time to Treatment, TTT), oder eine logische Verknüpfung von Zielen aus dem Navigationsraum. Eine solche Verknüpfung ist zum Beispiel auch die Bedingung, "zumindest eine der beiden Speicheldrüsen muss die Strahlentherapie überstehen, die linke oder rechte Speicheldrüse, L-Parotid oder R-Parotid".

Bei den Punkten im Navigationsraum, deren Kriterien zur Navigation auf Bedienhilfen aufgetragen werden können, beispielsweise auf Schiebereglern (folgend "Slider" genannt), ist eine quasi-stetige Navigation möglich. Die aufgetragenen Punkte aus dem Speicher ermöglichen zwischen benachbarten Punkten eine Interpolation von Zwischenwerten, die nur während der Navigation temporär gespeichert werden.

Zielachsen mit Kriterien aus dem Navigationsraum, bei denen die besagte quasi-stetige Navigation möglich ist, können auch im Entscheidungsraum dargestellt werden. Das soll kein Widerspruch zu Merkmal (d) sein, wie oben dargelegt.

Die Navigation im Navigationsraum und die Filterung im Entscheidungsraum werden für den Benutzer durch Darstellung auf einem Sichtgerät, wie Bildschirm, Tablett oder Projektion auf einen Screen oder Leinwand möglich, wobei eine kontinuierlich geführte Interaktion zwischen Mensch und Maschine Grundlage der Entscheidungsfindung wird, oder werden soll. Durch die Navigation erfährt der Benutzer interaktiv sichtbar die von ihm eingestellten Änderung der Kriterien in seinen Plänen, auch mit Folgen auf die anderen Kriterien. Durch die Filterung im Entscheidungsraum wird ihm in gleicher Weise "spürbar sichtbar" gemacht, welche Therapievorschläge verbleiben, wenn der Arzt durch seine binäre Auswahl vorhandene Therapievorschläge ablehnt oder ausblendet, insbesondere auf zumindest einigen Zielachsen ganze Bereiche abgewählt, im Sinne einer Filterung.

Bevorzugt erfolgt die binäre Auswahl im Entscheidungsraum durch Vorgabe eines ganzen Bereichs, der ausgefiltert wird. Der ganze Bereich wird festgelegt durch Vorgabe eines Restriktors. Links oder rechts von diesem Restriktor wird der ganze Bereich bis zum Ende der dargestellten Bedienhilfe abgewählt oder ausgefiltert.

Als Bedienhilfe eignen sich längs erstreckende Bedienhilfen, insbesondere die genannten "Slider", und auch drehend arbeitende Bedienhilfen. Bei Slidern gibt es einen Anfang und ein Ende der Bedienstrecke oder der Schiebestrecke, und einen Bedienknopf (als Handle), der visuell dargestellt sein kann, mit dem die Einstellung vorgenommen wird.

Bevorzugt ist der Bedienknopf der Restriktor (auch "Handle" genannt). Wird er von einem Ende der Schiebestrecke, seiner Ruhelage, verschoben, werden alle Lösungen im Entscheidungsraum ausgefiltert, die zwischen seiner Position, an welcher das Handle losgelassen wird oder platziert wird, also "gesetzt wird", und dem Ende der Schiebestrecke ausgefiltert. Dies gilt für beide Seiten des Sliders.

Bevorzugt können unterschiedliche Bereiche auf unterschiedlichen Zielachsen im Entscheidungsraum vorgegeben und damit ausgefiltert werden.

Bevorzugt können Zielachsen im Entscheidungsraum gruppiert werden. Eine Gruppierung oder Priorisierung der Ziele bietet sich an für eine Verbesserung der Übersichtlichkeit und Geschwindigkeit des Auffindens eines bestmöglichen Therapieplans.

Dies zum Beispiel wie folgt ...
notwendige Ziele (must have goals)
wichtige Ziele (important goals)
mit der Navigation nicht erfassbare Ziele (in-feasible goals).

Im Entscheidungsraum kann eine Gruppe von Technologien abgebildet werden, wobei jede Technologie für eine Therapiemaschine steht, beispielsweise ein Maschine, die mit Protonen arbeitet, oder eine solche, die mit Elektronen arbeitet oder eine weitere, die mit Photonen arbeitet. Auch Schwer-Ionen können als Strahlenteilchen in einer noch weiteren Maschine genutzt werden. In dieser dargestellten Gruppe kann jede Technologie angewählt oder abgewählt werden, was auch einer Filterung entspricht.

Therapiepläne einer abgewählten Technologie werden in allen Slidern nicht mehr dargestellt.

Auch können Varianten innerhalb einer Technologie als eine separate Gruppe dargestellt werden, beispielsweise verschiedene Einstrahlkonfigurationen oder andere technische Parameter einer Photonenbehandlung.

Im Entscheidungsraum können zusätzlich zu einer grafischen Visualisierung der nicht ausgewählten Planungsvorschläge entweder die noch verbliebenen optisch hervorgehoben werden, oder die ausgefilterten Planungsvorschläge optisch herausgenommen werden. Dies kann beispielsweise durch Verändern einer Grauschattierungen erfolgen, durch Farbveränderung, oder durch Schraffur- oder Strukturveränderungen, bevorzugt entlang des Sliders.

Punkte aus zwei verschiedenen Navigationsräumen können in einem gemeinsamen Entscheidungsraum abgebildet werden. Diese Option ermöglicht den Vergleich grundsätzlich unterschiedlicher Therapien, z.B. Chemotherapie und Strahlentherapie, die grundsätzlich verschiedene Navigationsräume aufweisen, die aber nach z.B. Risiko und Erfolg bewertet werden können (das wären zwei klinische Ziele im Entscheidungsraum). Eine zusätzliche Funktion ist eine Scoring Funktion, mit der aus den noch verbliebenen Planvorschlägen bestimmte hervorgehoben werden. Dies kann durch Markierung auf den Zielachsen geschehen, beispielsweise durch einen Marker oder einen Keil, wobei die Scoring Funktion bei mehreren verfügbaren Technologien für jede Technologie nur einen Plan vorschlägt, insbesondere den aus Sicht der Scoring Funktion bestmöglichen Planvorschlag. Durch Verändern der ausgefilterten Planvorschläge können entweder ganze Technologien abgewählt werden, also ausgefiltert werden, oder auf einem Slider nur Abschnitte von noch dargestellten Lösungen abgewählt oder ausgefiltert werden. Letzteres betrifft dann nicht die gesamte Technologie und die Scoring Funktion ergänzt eine neue Markierung für die Technologie, wenn die gerade dargestellte bestmögliche Planung durch Setzen des Restriktors ausgefiltert wird.

Der numerische Bereich der Lösungsmenge für ein Kriterium kann im Slider angezeigt werden. Diese Bereichsdaten werden aktualisiert, wenn der User den Filter auf die Lösung anwendet (z. B. über einen Filter-Restriktor).

Der Bereich kann auf zwei Arten angezeigt werden.
(1) Die numerischen Werte für das Minimum und Maximum werden innerhalb des Sliders angezeigt
(2) Eine Farbschattierung wird innerhalb des Sliders angezeigt, die den verfügbaren/gefilterten Bereich des Kriteriums anzeigt.

Die optimale(n) Lösung(en) können als besagte Markierungen entlang des Sliders angezeigt werden. Es kann der numerische Wert für alle optimalen Lösungen oder nur der einer globalen Lösung angezeigt werden.

Die Scoring-Funktion ermittelt z.B. eine nach Priorität gewichtete Punktzahl oder eine lexikografische Erfüllung von Anspruchsniveaus (oder beides). Die Scoring-Funktion kann durch das Setzen von Prioritäten für Entscheidungskriterien konfiguriert werden.

Eine Struktur, die GUI genannt wird, auf einem Display, einem Tablet, einem Sichtschirm oder einer andern "Darstellungseinrichtung" zum sichtbaren Kenntlichmachen einer funktionalen Steuerung umschreibt Anspruch 22. Er ist keine bloße Widergabe von Information, sondern beinhaltet die Funktionalität, einem Planer/User das Verändern eines Plans im Wege der Planfindung "greifbar" und sichtbar zu machen. Jeder dargestellte Punkt oder Slider steht für technische Einstellgrößen, die im Maschinenraum für Dosis, Dosismittelwert (allgemein: als mathematische (konvexe) Norm der Dosis) oder zumindest einen Einstrahlwinkel eines Strahls oder temporären Strahlenbündels stehen. Es sind ohne Zweifel technische Werte, ja sogar Messwerte oder messbare Werte, die von übergeordneten Vorgaben definiert werden.

Die technisch einsetzbare grafische Benutzeroberfläche (GUI) vermag das Auffinden eines Therapieplans unter Planvorschlägen zur Krebstherapie zu ermöglichen. Jede Lösung als Planvorschlag stellt einen Punkt in einem Navigationsraum (N) dar und bildet eine Paretolösung, die eine Vielzahl von Einstellparametern enthält (wie oben umschrieben), um in einem Maschinenraum die Einstellungen für abzugebende Strahlendosen eines Strahlentherapiegeräts (besser einer Strahlentherapiemaschine) der Krebstherapie festzulegen. Vorgesehen ist zumindest ein dargestellter Navigationsraum, der zumindest eine Paretofront aufweist, wobei eine Vielzahl von verfügbaren Planvorschlägen in einem physischen Speicher vorgehalten werden, die vorab berechnet wurden und zur Darstellung aus dem Speicher abgerufen oder ausgelesen werden. Die abgerufenen oder ausgelesenen Lösungen sind auf einem Display dargestellt und von dem die GUI benutzenden Benutzer mit Hilfe von Slidern für seine Navigation bedienbar. Ein weiterer, separat dargestellter Entscheidungsraum ist vorgesehen, in dem die navigierbaren Punkte aus dem Navigationsraum auf Zielachsen abgebildet sind, funktional ohne Möglichkeit, im Entscheidungsraum zu navigieren, indes nach Art eines Restriktors im Entscheidungsraum auf den Zielachsen als Slider ausschließlich binär auszuwählen, welche Planvorschläge als Therapiepläne nicht nutzbar sind oder nicht nutzbar sein sollen. Im Entscheidungsraum sind zumindest solche Zielachsen zur Filterung enthalten und dargestellt, die nicht im Navigationsraum (N) zur Navigation dargestellt sind.

Ausbildungen der GUI finden sich in den abhängigen Ansprüchen 23-26.

Ausführungsbeispiele erläutern die Erfindung ohne sie auf diese Beispiele zu beschränken.
Figur 1 veranschaulicht drei Räume, mit denen ein erstes Beispiel der Erfindung arbeitet. Es kann ein weiterer Raum N2 vorhanden sein, der nicht dargestellt ist, aber seine Kriterien können ebenso in den Entscheidungsraum D umgerechnet werden. D ist ein Entscheidungsraum, bevorzugt rötlich dargestellt. N ist der Navigationsraum. M ist der "Maschinenraum", bevorzugt in einem Blauton dargestellt.
Figur 2 zeigt einen ersten Status des Entscheidungsraums D, mit seinen Slidern.
Figur 3 veranschaulicht einen zweiten Status des Entscheidungsraums D, wobei eine (ganze) Technologie 90 abgewählt ist.

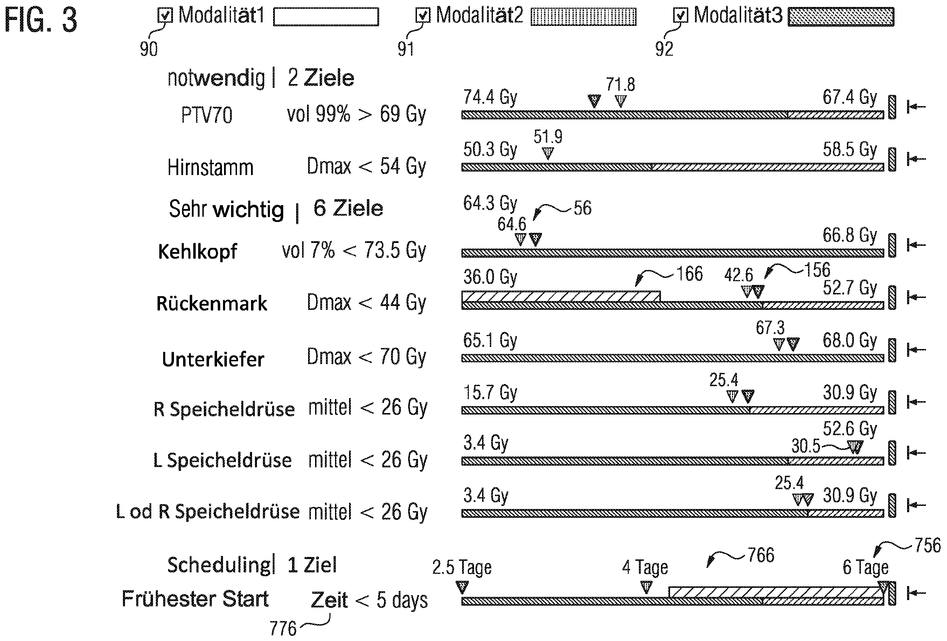

Ein nicht dargestelltes Beispiel erkärt aus Figur 3 den Entscheidungsraum D mit mehreren eingerückten Restriktoren, wobei z.B. das Stellglied 100 (ein Handle stellvertretend für auch andere Handles) aus einer (z.B. rechten) Ruhelage in eine Arbeitslage (nach z.B. links) verschoben wurde und dort gesetzt wurde (oder vice versa). Hier in der Arbeitslage bewirkt das Handle eine Restriktion, nimmt also Planvorschläge aus, oder filtert sie aus.

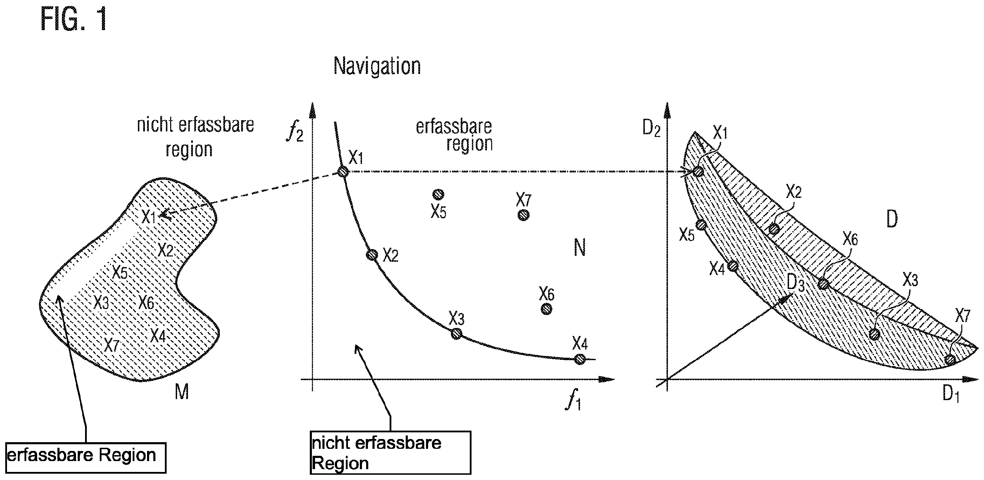

In der Figur 1 ist ein (erster) Navigationsraum N in der Mitte dargestellt. Links davon ist der Maschinenraum M (der Therapiemaschinen) und rechts davon der Entscheidungsraum D. Diese Darstellung ist eine Abstraktion und zeigt den Navigationsraum N mit seinen Lösungen x1 bis x7, die hier als Beispiel für alle verfügbaren Lösungen angesehen werden sollen. Diese Lösungen werden abgebildet in den Entscheidungsraum D mit gleichem Namen x1 bis x7 und bei einer Umsetzung zur Einstellung einer Therapiemaschine (nicht dargestellt) werden die hinter dem Punkt stehenden (also von ihnen repräsentierten) technischen Einstellungsparameter auf die Therapiemaschine übertragen.

Im Navigationsraum N ist die Pareto Funktion 200 zu sehen, rechts oberhalb sind die erreichbaren Therapievorschläge als Lösungspunkte zu sehen. Nicht dargestellt ist die Organisation der Punkte in einer für die Navigation auf Slidern geeigneten Form, diese steht aber hinter dem Navigationsraum N. Beispiele sind einzelne Zielgrößen auf einzelnen Achsen, wobei jede Zielachse einen Slider darstellt. Auf diese Weise können, bei einer Kopplung der mehreren Slider über die Lösungsmenge Filterungen vorgenommen werden. Das Verändern eines Handles, z.B. Sliders, verändert bei der Navigation auch die Positionen der anderen Slider, respektive deren Handles, z.B. Sliders. Letztere Bedienknöpfe sind im grafischen Bereich als Handle zu sehen und die allgemeine Formulierung ist die lang gestreckte Bedienhilfe.

Bei der Filterung ist es anders. Hier werden die auf einer Zieleachse ausgefilterten Planungsvorschläge auch auf anderen Zielachsen entfernt, sie bilden dort also nicht mehr eine verfügbare Menge von Paretolösungen. Das Ausfiltern erfasst damit alle dargestellten Werte auf Zielachsen, auch wenn nur einer der Zielwerte auf einer der Zielachsen ausgefiltert wird. Das Handle als Slider bekommt eine andere Funktion.

Im Entscheidungsraum ist die Punktewolke, welche aus dem Navigationsraum N dort abgebildet wird, unterschieden in zwei von verschiedenen Grauwerten unterlegten Zonen. Dies repräsentiert die Pareto Lösungen (dunkler) und die nicht Pareto Lösungen, die für die Lösungsfindung nicht berücksichtigt werden. Unter denen ist x2.

Anhand der im Entscheidungsraum abgebildeten Punkte kann die Score Funktion verstanden werden. Geht der User/Benutzer, bevorzugt ein Arzt, auf dem Kriterium D1 nach links und filtert er x3 aus, bildet die Score Funktion selbsttätig einen neuen Marker auf dem zugehörigen Slider, der x6 entspricht. Es wird für den User/Benutzer so empfunden, dass der Marker auf dem Slider "springt", von x3 auf x6 (ohne Zwischenstufen).

Die vom Punkt x1 weg gehenden Pfeile symbolisieren die Umrechnung in einen jeweils anderen Raum.
(a) Von dem Navigationsraum N wird der Punkt x1 mit seiner Einstellung in den Maschinenraum M umgerechnet, dort auch als x1 bezeichnet. Er repräsentiert die Einstellung, oder eine Einstellung einer Therapiemaschine. Der gleiche Punkt x1 wird umgerechnet in den Punkt x1 im Entscheidungsraum D. Dort steht er als klinisches Ziel zur Verfügung. Die Darstellung mit den Punkten repräsentiert die in den folgenden Figuren verwendete Darstellung mit den Slidern.
(b) Ein Beispiel einer Umrechnung vom Navigationsraum N in den Entscheidungsraum D ist wie folgt umschrieben.

Wir betrachten den Fall der Einhaltung mindestens eines von zwei Zielen. Die Ziele sind konvex (also aus dem Navigationsraum N), wobei das übergeordnete Ziel mindestens eines davon zu erfüllen nur im Entscheidungsraum D sinnvoll behandelbar ist.

Ein Ziel, genannt ZL, ist die Begrenzung der durchschnittlichen Dosis unter 26 Gray (Gy) in der linken Speicheldrüse (L-Parotid) und ein weiteres Ziel ZR ist die gleiche Begrenzung in der rechten Speicheldrüse (R-Parotid). Mathematisch formuliert sind diese Ziele wie folgt $ZL : \frac{1}{{n}_{SL}} {\sum }_{i ∈ {V}_{SL}} {d}_{i} \leq 26 Gy$ und $ZR : \frac{1}{{n}_{SR}} {\sum }_{i ∈ {V}_{SR}} {d}_{i} \leq 26 Gy$

Wobei die Dosis hier wie allgemein üblich als eine Reihe von Werten *dᵢ* dargestellt wird und der Index i für ein kleines Volumenanteil ("Voxel") im Körper des Patienten darstellt - typischerweise ein kleiner Würfel von 3mm Kantenlänge. Die Indizes der Voxel die zur linken (rechten) Speicheldrüse gehören werden mit *V_{SL}* (*V_{SR}*)dargestellt - es ist eine Liste von Voxelindizes. Die Summe *∑*_{*i*∈}*_{VSL} dᵢ* stellt somit die Gesamtsumme der Dosis in der linken Speicheldrüse dar (die rechte analog). *n_{SL}* ist die Gesamtzahl der Voxel in der linken Speicheldrüse (*n_{RL}* analog) durch welche die Summe geteilt wird, um die Durchschnittsdosis zu erhalten. Beide Ziele allein genommen sind konvex und im Navigationsraum N be- oder abhandelbar.

Die Forderung nach der Schonung mindestens einer Speicheldrüse ist nun ein übergeordnetes Ziel das wir ZS nennen und ist wie folgt formuliert: $ZS : ZL ∨ ZR$

Wobei "∨" das aus der boolschen Logik bekannte Zeichen für "oder" darstellt. Als Formel ausgedrückt lässt sich ZS in äquivalenter Weise wie folgt beschreiben: $ZS : χ \left(\frac{1}{{n}_{SL}}{\sum }_{i ∈ {V}_{SL}} {d}_{i} \leq 26 Gy\right) + χ \left(\frac{1}{{n}_{SR}}{\sum }_{i ∈ {V}_{SR}} {d}_{i} \leq 26 Gy\right) \geq 1$

Wobei *χ*(*A*) eine nichtkonvexe Funktion ist die für ein beliebiges Ereignis *A* den Wert 1 liefert wenn *A* wahr ist und 0 liefert wenn *A* nicht wahr ist. Die Forderung in der obigen Gleichung für ZS ist also also erfüllt wenn mindestens eine der beiden Speicheldrüsenziele erfüllt ist.

Auf diese Weise kann, an einem Beispiel gezeigt, die Umrechnung vom Navigationsraum N in den Entscheidungsraum D erfolgen. Für andere konvexe Ziele (also aus dem Navigationsraum N) erfolgt es entsprechend.

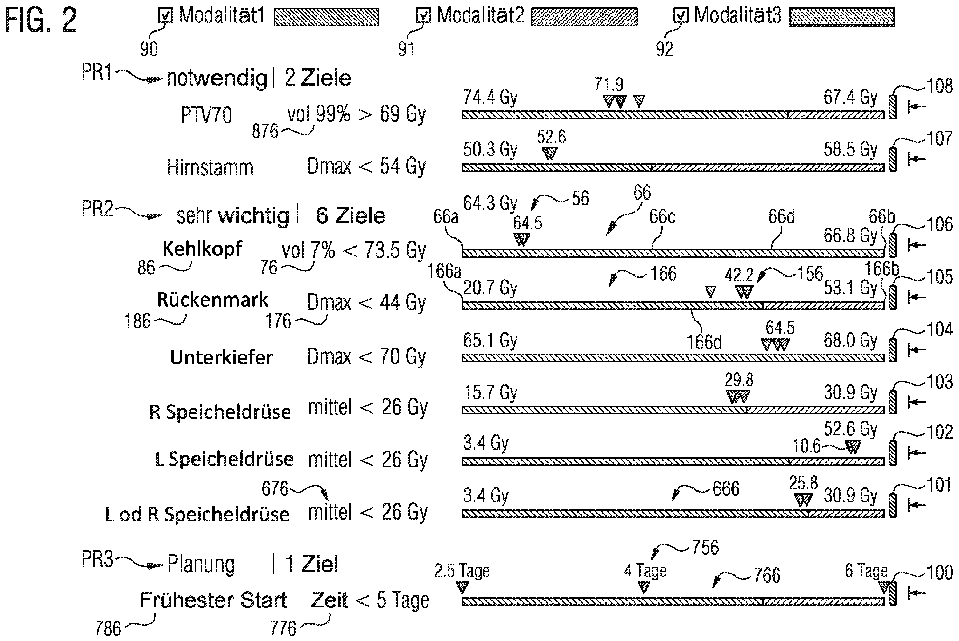

Figur 2 veranschaulicht einen mit Slidern versehenen Entscheidungsraum D, der auf einem Sichtschirm dargestellt ist. Als Sichtschirm soll das Blatt angesehen werden. Andere Formen der Darstellung sind ein Display oder ein berührungsgesteuertes Tablet. Auch möglich ist ein Projektor, der ein Lichtsignal auf eine Projektionsfläche projiziert. Die Benutzung erfolgt dabei von einem zweiten Display aus, das per Finger mit Wipe und Tap (Swipe/Wischen und Tippen) oder per Mauszeiger bedienbar ist.

Auf dem Display der Figur 2 ist in der Prioritätsgruppe PR3 eine Zielachse als Slider 766 aufgetragen, die nicht im Navigationsraum N vorhanden war. Mit ihr wurde nicht navigiert. Zusätzlich zu diesem Slider als Zielachse 766 sind sechs weitere Slider als Zielachsen in der Prioritätsgruppe PR2 vorgesehen und parallel untereinander aufgetragen. Oberhalb sind zwei Zielachsen angeordnet, die der Prioritätsgruppe PR1 angehören. Ganz oben sind drei Technologien repräsentiert, welche drei reale Therapiemaschinen darstellen, nach der Art der von Ihnen jeweils abgegebenen Strahlung. Im Beispiel kann angenommen werden, dass es Protonen, Elektronen und Photonen sind, die diese drei Technologien 90, 91 und 92 darstellen, bevorzugt 90 in einem dunklen Rotton, 91 in einem Gelbton und 92 in einem Blauton. Die Unterschiede sind mit Schraffuren hier symbolisiert, können auch als verschiedene Farben im o.g. Sinn gestaltet sein.

Jede der Technologien kann angewählt und abgewählt werden. Dargestellt ist der angewählte Zustand. Es ist also keine Technologie ausgefiltert.

Die Reihenfolge der Anordnung ist keine Beschränkung auf dieses Beispiel. Es kann ebenso eine andere Anordnung vorgesehen sein, die Technologien rechts oder unten und die Slider können vertikal ausgerichtet sein.

Der Aufbau jedes Sliders ist gleich, nur steht er für ein jeweils anderes Organ der Krebstherapie.

Drei dieser Slider sollen erläutert werden. Auf dem Slider 66, der eine Zielachse definiert, ist die erreichbare Strahlendosis in Gray (Gy) mit einem Balken veranschaulicht. Die Strahlendosis reicht von 64,3 Gy bis 66,8 Gy. Diese beiden Werte sind an den Endlagen 66a und 66b des Sliders 66 gegeben. Auf der rechten Endlage liegt der Restriktor 106. Nach der Funktion kann dieser Restriktor auf dem Slider und damit auf der Zielachse nach links bewegt werden. Wo er gesetzt wird, übt er eine Filterfunktion aus, wonach alle rechts von ihm liegenden Werte und damit alle zugehörigen Lösungen, die auf den Zielachsen abgebildet sind, ausgefiltert werden.

Die Filterfunktion kann durch dunkleres Darstellen des ausgefilterten Abschnitts im gesamten Balken 66c dargestellt werden. Der Slider 66 steht für das Zielvolumen "Kehlkopf", welches mit 86 benannt ist. Die zugehörige Dosisvorgabe ist bei 76 angegeben. Der Kehlkopf soll weniger als 73,5 Gy Strahlung erhalten, in einem Volumenanteil von 7%. Nachdem der dem Slider parallele schmale Balken 66d mit allen Lösungen diese Vorgabe erfüllt, ist der Slider mit einer dies kennzeichnenden Farbe neben dem Balken 66c gekennzeichnet.

Andere Kennzeichnungen sind ebenfalls möglich, eingangs war erwähnt, dass es eine unterschiedliche Schraffur oder eine unterschiedliche Struktur oder eine unterschiedliche Farbe sein kann.

Auch dargestellt ist die Markierung 56, welche für alle drei Technologien eine Planung als bestmögliche Planung aus der Scoring Funktion hervorhebt und mit einem Dreieck kennzeichnet. Die jeweilige Farbe oder Schattierung oder Struktur kann der jeweiligen Technologie 90, 91 und 92 angepasst sein. Zusätzlich kann der Strahlen-Dosiswert für das Organ der Zielachse angegeben sein, hier 64,6 Gy.

Angelehnt an den Slider 66 soll der Slider 166, der darunter liegt, erläutert werden. Er betrifft als medizinisches Volumen das Rückenmark, welches an der Position 186 gekennzeichnet ist. An der Position 176 ist vorgegeben, dass die maximale Dosis <44 Gy sein darf. Der zum Balken 166c parallele Balken 166d hat zwei Farben, ist also nur bis zum Wert 44 Gy in der einen Farbe und bei Werten darüber in der anderen Farbe. Das bestimmt die Vorgabe 176, die diesen Wert vorgibt. Ein Verschieben des Restriktors 105 auf den Übergang zwischen den beiden Farben würde dazu führen, dass die ohnehin nicht der Vorgabe 176 entsprechenden Lösungen ausgenommen werden, also herausgefiltert werden.

Die Scoring Funktion zeigt auch bei diesem Slider 166 für jede Technologie einen bestmöglichen Wert an, dies ist der Abschnitt 156. Die Markierung entspricht jeweils der Farbe, der Struktur oder der Schraffur der Kennzeichnung der Technologie 90, 91 oder 92.

Der Restriktor 105 kann auch über den Wert 44 Gy zurück verschoben werden, um alle höheren Dosiswerte auszunehmen. Er muss sich nicht nur auf die Vorgabe 176 einstellen lassen, sondern kann mehr oder weniger zulassen, also weniger oder mehr der Lösungen ausnehmen.

In der ersten Gruppe PR1 ist ein Tumorvolumen PTV70 als medizinisches Volumen angegeben. Dieses soll nach der Vorgabe in 99 % Volumen mit einem Dosiswert von mehr als 69 Gy beaufschlagt werden. Darunter liegt der Slider für den Hirnstamm. Hier lautet die Vorgabe, dass die maximale Dosis <44 Gy sein soll.

Bei den beiden beschriebenen Slidern, die unter die Gruppe PR1 fallen, ist eine große Relevanz gegeben. Durch Einstellen der Restriktoren 107, 108 auf Positionen im Slider und dortiges Setzen können die Lösungen ausgenommen werden, welche die Vorgabe nicht erfüllen, was in dem parallelen Balken, entsprechend 166d, farblich gekennzeichnet sein kann. Nachdem alle Slider über die Lösungsmenge miteinander verkoppelt sind, ist das Ausnehmen von Lösungen durch einen Restriktor automatisch auch das Ausnehmen von den zugehörigen Werten auf den anderen Slidern. Wird eine Lösung ausgenommen, fällt sie für alle Slider heraus.

Ein Slider 766 soll noch erläutert werden. Er trägt keine solche Zielachse, die im Navigationsraum zur Navigation vorgesehen war oder ist. Es ist die Angabe eines frühestmöglichen Beginns einer Behandlung. Die Vorgabe lautet, dass die Zeit <5 Tage sein soll und die Eigenschaft 786 nennt den Parameter des frühestmöglichen Beginns der Behandlung. Diese Zielachse ist einer quasi-stetigen Veränderung nicht zugänglich, sie wird also für eine Navigation im Navigationsraum N nicht benutzt.

Ein weiterer Slider 666 soll auch erläutert werden. Er gibt die Zielachse an, bei der eine logische Verknüpfung von navigierbaren Zielen aus dem Navigationsraum stattfindet. Es soll die linke oder die rechte Speicheldrüse die Strahlentherapie überstehen. Dies wird mit der Vorgabe 676 eingestellt, wonach der Mittelwert <26 Gy sein soll. Die beiden Speicheldrüsen sind auch über die beiden Zielachsen darüber jeweils individuell abgebildet und für eine Filterung verfügbar. Sowohl für die linke Speicheldrüse wie auch für die rechte Speicheldrüse soll der Mittelwert <26 Gy sein

Figur 3 veranschaulicht das Herausnehmen, oder das Filtern der Technologie 90. Diese wird vom User ausgenommen, woraufhin er im Entscheidungsraum den besten Bereich für das Rückenmark verliert. Dies kennzeichnet ein dunklerer grauer Balkenabschnitt im Slider 166, der nicht mehr verfügbar ist. Entsprechend reagiert auch der Slider 766, dem ein rechter Abschnitt fehlt, durch den heraus genommenen Balkenabschnitt, in dem die sechs Tage liegen, welche ohnehin das vorgegebene Kriterium verletzen. Der User hat die Entscheidungskriterien damit verschärft.

Das Einrücken mehrerer "Handles" (Einstellorgane) 100, 101 105, 107 und 108 der Slider ist vorstellbar. Hier hat der User viele Kriterien verschärft. Dabei fallen nicht nur ganze Bereiche rechts von dem jeweiligen Handle heraus, sondern auch durch die Kopplung der Slider über die vorhandene Lösungsmenge mögliche Lösungen auf der anderen Seite des Handles heraus, werden also auch ausgefiltert.

Von den beiden Handles 107 und 108 wird der Bereich rechts ausgefiltert, der ohnehin nicht der Vorgabe entspricht, wonach in 99% des Volumens mindestens 69 Gy für das Target PVT70 gegeben sein muss und die maximale Dosis im Hirnstamm 54 Gy nicht überschreiten darf.

Die Handles 107 und 108 sind auch an den Slidern vorgesehen, bei denen als Score Funktion die Modalität als Technologie 91 einen Dosiswert von 71,4 bzw. 52,2 vorschlägt. Dieser Dosiswert bleibt nach Abwahl einer Vielzahl von anderen Lösungsvorschlägen, dargestellt durch die dunkelgrauen Balken 866c und 966c übrig. Er kam, wie gesagt, nicht durch Navigation zustande, sondern durch Abwahl eine Vielzahl von Lösungsvorschlägen, durch Setzen der Restriktoren 108,107,105 und 101 sowie 100.

Das klinische Ziel 876 kann dabei erfüllt werden. Eine physikalische Bewertung eines Dosismittelwertes findet sich im Slider 966 mit der Vorgabe, dass der Hirnstamm einen Dosiswert von <54 Gy erhalten darf. Die Zielvorgabe 76 ist erneut ein klinisches Ziel, da weniger als 7% des Volumens des Kehlkopfes eine Dosis <73,5 Gy erhalten dürfen. Alle aufgetragenen Werte auf dem Slider 66 erfüllen dieses Kriterium.

In den Figuren 2, 3 sind die klinischen Ziele gruppiert. Zwei Ziele sind den notwendigen Zielen zugeordnet. Sechs Ziele sind zu den wichtigen Zielen zugeordnet und ein klinisches Ziel ist in die Prioritätsgruppe PR3 eingeordnet. Es hängt von dem Benutzer/User, z.B. Arzt ab, ob der Patient dieses Ziel als wichtig empfindet, daher hat es eine Art gesonderten Status.

Nicht dargestellt ist das Beispiel, bei dem ein zweiter Navigationsraum N2 vorhanden ist, der eine grundsätzlich andere Therapie repräsentiert, beispielsweise eine Chemotherapie. Die dort angegebenen Kriterien des Navigationsraums werden mit einer alternativen Umrechnung in die klinischen Ziele des Entscheidungsraums umgerechnet, und zwar desselben Entscheidungsraums D, in den auch die physikalischen Werte oder Bewertungen der Volume of Interest aus dem Navigationsraum N in die klinischen Ziele des Entscheidungsraums D umgerechnet werden und dargestellt werden, um diese beiden grundsätzlich unterschiedlichen Therapien im Entscheidungsraum D zu vergleichen.

## Patentansprüche

1. **Verfahren** zum **Auffinden und Festlegen** eines Therapieplans unter einer Vielzahl von Planvorschlägen zur Krebstherapie, wobei jeder Planvorschlag als Lösung einen Punkt in einem Navigationsraum (N) darstellt, der eine Vielzahl von Einstellparametern enthält und in einem Maschinenraum (M) eine Einstellung für abzugebende Strahlendosen einer Strahlentherapiemaschine der Krebstherapie festlegt;
(a) der Navigationsraum (N) zumindest eine Paretofront (200) enthält, hinter oder auf welcher verfügbare Behandlungspläne als mögliche Lösungen liegen und unter denen von einem Benutzer navigiert wird, wobei eine erste Vielzahl von verfügbaren Lösungen in einem Speicher vorgehalten werden, die als Planvorschläge vorab berechnet wurden und zur Darstellung aus dem Speicher abgerufen oder ausgelesen werden;
(b) die abgerufenen oder ausgelesenen Planvorschläge auf einem Display dargestellt und von dem Benutzer für eine Navigation benutzt werden, wobei sich unter den Punkten eine große Anzahl von pareto-optimalen Lösungen mit physikalischen Werten oder Bewertungen von Dosiswerten findet, aber auch solche Lösungen dargestellt werden, die fast-pareto-optimal sind, in einem Toleranzbereich von höchstens ±5% vom ParetoOptimium entfernt;
(c) womit eine große erste Punktemenge mit den physikalischen Werten oder Bewertungen von Dosiswerten geschaffen wird, diese im Speicher abgelegt und daraus eine zweite Punktemenge berechnet wird, die klinische Ziele definiert;
(d) wobei ein Entscheidungsraum (D) vorgesehen ist und dargestellt wird, in dem zumindest die klinischen Ziele auf Zielachsen (76,876,776) aufgetragen werden;
ohne Möglichkeit, im Entscheidungsraum (D) auf den Zielachsen der klinischen Ziele zu navigieren, indes nach Art von Restriktoren im Entscheidungsraum binär auszuwählen, welche Planvorschläge als Therapiepläne nicht benutzt werden und dadurch einen gewünschten Therapieplan aus der Vielzahl von Planvorschlägen aufzufinden und festzulegen.

2. Verfahren nach Anspruch 1, wobei im Entscheidungsraum (D) auch solche Zielachsen (166) mit ihren Kriterien (176) aufgetragen sind, die physikalische Werte von Dosiswerten enthalten, insbesondere für ein Volume of Interest einen zugehörigen Dosismittelwert als eine mathematische (konvexe) Norm der Dosis angeben.

3. Verfahren nach Anspruch 1, wobei unter den im Entscheidungsraum (D) aufgetragenen Zielachsen eine Zielachse (766) ist, die als passives Ziel eine Zeitspanne für eine von mehreren Therapiemaschinen angibt, zu der eine Behandlung auf dieser Therapiemaschine möglich ist oder vor der eine Behandlung auf dieser Therapiemaschine nicht möglich ist.

4. Verfahren nach Anspruch 1 oder 3, wobei unter den im Entscheidungsraum (D) aufgetragenen Zielachsen eine Zielachse (666) ist, die eine logische Verknüpfung von Zielen aus dem Navigationsraum (N) darstellt, insbesondere eine Bedingung
zumindest eine von zwei Speicheldrüsen muss die aufgefundene und festgelegte Strahlentherapie überstehen;
oder
zumindest ein Sehapparat von zwei Sehapparaten muss die aufgefundene und festgelegte Strahlentherapie überstehen, wobei ein Sehapparat aus Sehnerv und Auge besteht;
oder
zumindest ein Organ von doppelt vorhandenen Organen, wie Lunge, muss die aufgefundene und festgelegte Strahlentherapie überstehen.

5. Verfahren nach einem der vorigen Ansprüche, wobei der Toleranzbereich um das Paretooptimum durch Rechenungenauigkeit, Lageungenauigkeit, Modellfehler oder numerische Ungenauigkeit vorgegeben wird.

6. Verfahren nach einem der vorigen Ansprüche, wobei auf zumindest einer der Zielachsen mit den klinischen Zielen im Entscheidungsraum (D) ein ganzer Bereich zusammenhängend abgewählt wird, veranlasst durch ein Setzen eines zu dieser Zielachse gehörenden Restriktors (100, 101).

7. Verfahren nach Anspruch 6, wobei ein weiterer Bereich zusammenhängend abgewählt wird, veranlasst durch ein Setzen eines zu einer weiteren Zielachse im Entscheidungsraum (D) gehörenden weiteren Restriktors (100, 101), wodurch bevorzugt unterschiedliche Bereiche auf unterschiedlichen Zielachsen im Entscheidungsraum (D) ausgefiltert werden.

8. Verfahren nach einem der vorigen Ansprüche, wobei mehrere Zielachsen im Entscheidungsraum gruppiert werden (PR1, PR2, PR3).

9. Verfahren nach Anspruch 8, wobei die Gruppierung notwendige Ziele, wichtige Ziele und mit der Navigation im Navigationsraum nicht erfassbare Ziele umfasst.

10. Verfahren nach einem der vorigen Ansprüche, wobei im Entscheidungsraum (D) eine Gruppe von unterschiedlichen Technologien (91,92,93) abgebildet wird.

11. Verfahren nach Anspruch 10, wobei eine Scoring Funktion Marker (56,156) für bestimmte Planvorschläge automatisiert setzt, insbesondere nur einen, bevorzugt bestmöglichen Planvorschlag für eine jeweilige Technologie.

12. Verfahren nach Anspruch 11, wobei der Marker (56,156) an einem Slider (66,166) gesetzt wird, der einen Restriktor besitzt.

13. Verfahren nach einem der vorigen Ansprüche, wobei eine Rekombination der im Entscheidungsraum (D) dargestellten Ziele zurück in den Navigationsraum (N) rechnerisch nicht möglich ist.

14. Verfahren nach einem der vorigen Ansprüche, wobei die erste Punktemenge mit den physikalischen Bewertungen oder Werten von Dosiswerten größer als 1.000 ist.

15. Verfahren nach einem der vorigen Ansprüche, wobei die berechnete zweite Punktemenge, die jeweils klinische Ziele aufträgt, in den Entscheidungsraum (D) umgerechnet wird, indes nur diejenigen Punkte, die im Entscheidungsraum (D) pareto-optimal sind, dargestellt werden.

16. Verfahren nach einem der vorigen Ansprüche, wobei Punkte aus der ersten Punktemenge mit den physikalischen Werten oder Bewertungen von Dosiswerten auf Slidern (66,166) im Entscheidungsraum (D) aufgetragen werden, wobei jeder Slider einen Restriktor (106,105) aufweist.

17. Verfahren nach einem der vorigen Ansprüche, wobei die erste Punktemenge mit physikalischen Werten oder Bewertungen von Dosiswerten durch Interpolationen zwischen den abgerufenen oder ausgelesenen Planvorschlägen bei der Navigation des Benutzers vergrößert wird, diese vergrößerte erste Punktemenge im Speicher abgelegt und daraus die zweite Punktemenge berechnet wird, welche klinische Ziele definiert.

18. Verfahren nach einem der vorigen Ansprüche, wobei die Berechnung der zweiten Punktemenge keine 1:1 Beziehung zu der ersten Punktemenge aufweist.

19. Verfahren nach einem der vorigen Ansprüche, wobei in der ersten Punktemenge mit physikalischen Werten oder Bewertungen von Dosiswerten Zielgrößen sind, die eine Dosis-Untergrenze, eine Dosis-Obergrenze oder einen Dosis-Mittelwert darstellen.

20. Verfahren nach einem der vorigen Ansprüche, wobei die erste Punktemenge während der Navigation im Navigationsraum (N) nach konvexen Funktionen berechnet wird.

21. Verfahren nach einem der vorigen Ansprüche, wobei in der ersten Punktemenge mit physikalischen Werten oder Bewertungen von Dosiswerten Dosismittelwerte enthalten sind, wobei diese je nach Organ unterschiedlich berechnet werden, für serielle Organe nach der Maximum Norm, für parallele Organe nach dem arithmetischen Mittel.

22. **Technisch einsetzbare grafische Benutzeroberfläche** zum Auffinden eines Therapieplans unter Planvorschlägen zur Krebstherapie, wobei jede Lösung als Planvorschlag einen Punkt in einem Navigationsraum (N) darstellt und eine Paretolösung bildet, die eine Vielzahl von Einstellparametern enthält, um in einem Maschinenraum (M) die Einstellungen für abzugebende Strahlendosen eines Strahlentherapiegeräts der Krebstherapie festzulegen;
(a) mit einem Navigationsraum (N), der zumindest eine Paretofront (200) aufweist, wobei eine Vielzahl von verfügbaren Planvorschlägen in einem Speicher vorgehalten werden, die vorab berechnet wurden und zur Darstellung aus dem Speicher abgerufen oder ausgelesen werden; wobei die abgerufenen oder ausgelesenen Lösungen auf einem Display dargestellt werden und von dem Benutzer mit Hilfe von Slidern für seine Navigation benutzbar sind;
(c) wobei ein weiterer, separat dargestellter Entscheidungsraum (D) vorgesehen ist, in dem die navigierbaren Punkte aus dem Navigationsraum auf Zielachsen (66,166,266) abgebildet werden, ohne Möglichkeit, im Entscheidungsraum (D) zu navigieren, indes nach Art eines Restriktors im Entscheidungsraum auf den Zielachsen als Slider binär auszuwählen, welche Planvorschläge als Therapiepläne nicht nutzbar sind oder nicht nutzbar sein sollen;
(d) Im Entscheidungsraum (D) zumindest solche Zielachsen (766) zur Filterung enthalten und dargestellt sind, die nicht im Navigationsraum (N) zur Navigation dargestellt sind.

23. Grafische Benutzeroberfläche nach Anspruch 22, wobei Zielachsen mit Kriterien aus dem Navigationsraum (N), auf denen eine quasi-stetige Navigation möglich ist, auch im Entscheidungsraum (D) dargestellt sind, insbesondere als Slider (766,666).

24. Grafische Benutzeroberfläche nach einem der vorigen Ansprüche 22 oder 23, wobei Zielachsen im Entscheidungsraum gruppiert dargestellt sind (PR1, PR2, PR3).

25. Grafische Benutzeroberfläche nach Anspruch 22, wobei die Gruppierung notwendige Ziele, wichtige Ziele und mit der Navigation nicht erfassbare Ziele umfasst.

26. Grafische Benutzeroberfläche nach einem der vorigen Ansprüche 22 bis 25, wobei im Entscheidungsraum (D) eine Gruppe von nicht gleichen Technologien (91,92,93) abgebildet sind, die im Maschinenraum (M) unterschiedlichen Strahlentherapie-Maschinen entsprechen, insbesondere entsprechend der Art ihrer Strahlen.

## Claims

1. **Method for identifying and determining** a treatment plan from a plurality of proposed plans for cancer treatment, wherein each proposed plan represents, as a solution, a point in a navigation space (N) comprising a plurality of adjustment parameters and determines, in a machine space (M), a setting for the radiation doses to be delivered by a radiotherapy machine used in cancer treatment;
(a) the navigation space (N) contains at least one Pareto front (200), behind or on which available treatment plans lie as possible solutions and amongst which a user navigates, wherein a first plurality of available solutions are stored in a memory; these solutions have been calculated in advance as treatment plan proposals and are retrieved or read from the memory for display;
(b) the retrieved or read-out plan proposals are displayed on a screen and used by the user for navigation , whereby the points include a large number of Pareto-optimal solutions with physical values or dose value assessments, but also solutions that are nearly Pareto-optimal, lying within a tolerance range of no more than ±5 per cent from the Pareto optimum;
(c) thereby creating a large first set of points comprising the physical values or dose value assessments, storing these in memory, and calculating from them a second set of points that defines clinical objectives;
(d) whereby a decision space (D) is provided and displayed, in which at least the clinical objectives are plotted on objective axes (76, 876, 776);
without the possibility of navigating within the decision space (D) along the target axes of the clinical targets, but rather by making binary selections within the decision space, in the manner of restrictors, as to which plan proposals are not to be used as treatment plans, thereby identifying and determining a desired treatment plan from the multitude of plan proposals.

2. Method according to claim 1, wherein the decision space (D) also includes target axes (166) with their criteria (176) which contain physical values of dose, in particular specifying, for a volume of interest ( , a corresponding mean dose value as a mathematical (convex) norm of the dose.

3. Method according to claim 1, wherein, amongst the target axes plotted in the decision space (D), there is a target axis (766) which, as a passive target, specifies a time interval for one of a plurality of therapy machines during which treatment on that therapy machine is possible or prior to which treatment on that therapy machine is not possible.

4. A method according to claim 1 or 3, wherein, amongst the target axes plotted in the decision space (D), there is a target axis (666) which represents a logical combination of targets from the navigation space (N), in particular a condition
at least one of two salivary glands must survive the identified and specified radiotherapy;
or
at least one visual apparatus out of two visual apparatuses must survive the identified and defined radiotherapy, wherein a visual apparatus consists of the optic nerve and the eye;
or
at least one organ from a pair of organs, such as the lungs, must survive the identified and specified radiotherapy.

5. Method according to any of the preceding claims, wherein the tolerance range around the Pareto optimum is determined by computational inaccuracy, positional inaccuracy, modelling errors or numerical inaccuracy.

6. Method according to any of the preceding claims, wherein, on at least one of the target axes, an entire contiguous region containing the clinical targets in the decision space (D) is deselected, triggered by setting a restrictor (100, 101) associated with that target axis.

7. Method according to claim 6, wherein a further contiguous region is excluded, triggered by the setting of a further restrictor (100, 101) associated with a further target axis in the decision space (D), whereby different regions on different target axes in the decision space (D) are preferably filtered out.

8. Method according to one of the preceding claims, wherein a plurality of target axes are grouped in the decision space (PR1, PR2, PR3).

9. Method according to claim 8, wherein the grouping comprises necessary targets, important targets and targets that cannot be detected by navigation in the navigation space.

10. Method according to one of the preceding claims, wherein a group of different technologies (91, 92, 93) is mapped in the decision space (D).

11. Method according to claim 10, wherein a scoring function automatically assigns markers (56, 156) to specific plan proposals, in particular only one, preferably the best possible plan proposal for a respective technology.

12. Method according to claim 11, wherein the marker (56, 156) is set on a slider (66, 166) which has a restrictor.

13. Method according to any one of the preceding claims, wherein a recombination of the objectives represented in the decision space (D) back into the navigation space (N) is computationally impossible.

14. Method according to one of the preceding claims, wherein the first set of points comprises physical assessments or dose values greater than 1,000.

15. Method according to one of the preceding claims, wherein the calculated second set of points, each of which corresponds to a clinical target, is mapped onto the decision space (D), whilst only those points that are Pareto-optimal in the decision space (D) are displayed.

16. Method according to any one of the preceding claims, wherein points from the first set of points, with the physical values or assessments of dose values, are plotted on sliders (66, 166) in the decision space (D), each slider comprising a restrictor (106, 105).

17. Method according to any of the preceding claims, wherein the first set of points with physical values or assessments of dose values is expanded by interpolation between the retrieved or read-out plan proposals during the user's navigation; this expanded first set of points is stored in memory; and the second set of points, which defines clinical targets, is calculated therefrom.

18. Method according to one of the preceding claims, wherein the calculation of the second set of points does not have a 1:1 relationship with the first set of points.

19. Method according to one of the preceding claims, wherein the first set of points comprises physical values or assessments of dose values representing target parameters such as a lower dose limit, an upper dose limit or a mean dose value.

20. Method according to one of the preceding claims, wherein the first set of points is calculated during navigation in the navigation space (N) according to convex functions.

21. Method according to one of the preceding claims, wherein the first set of points comprising physical values or evaluations of dose values includes mean dose values, which are calculated differently depending on the organ: for serial organs according to the maximum norm, and for parallel organs according to the arithmetic mean.

22. **Technically implementable graphical user interface** for identifying a treatment plan from among proposed plans for cancer treatment, wherein each solution, as a treatment plan proposal, represents a point in a navigation space (N) and forms a Pareto solution containing a plurality of adjustment parameters for determining, in a machine space (M), the settings for the radiation doses to be delivered by a radiotherapy device used in cancer treatment;
(a) comprising a navigation space (N) which has at least one Pareto front (200), wherein a plurality of available treatment plan proposals are stored in a memory; these have been calculated in advance and are retrieved or read from the memory for display; the retrieved or read solutions are displayed on a screen and can be used by the user for navigation with the aid of sliders;
(c) whereby a further, separately displayed decision space (D) is provided, in which the navigable points from the navigation space are mapped onto target axes (66, 166, 266), without the possibility of navigating within the decision space (D), whilst, in the manner of a restrictor within the decision space, it is possible to select via sliders on the target axes which proposed plans are not usable or should not be used as treatment plans;
(d) The decision space (D) contains and displays at least those target axes (766) for filtering which are not displayed in the navigation space (N) for navigation purposes.

23. Graphical user interface according to claim 22, wherein target axes from the navigation space (N) on which quasi-continuous navigation is possible are also displayed in the decision space (D), in particular as sliders (766, 666).

24. Graphical user interface according to one of the preceding claims 22 or 23, wherein target axes are displayed in groups (PR1, PR2, PR3) within the decision space.

25. Graphical user interface according to claim 22, wherein the grouping comprises necessary targets, important targets and targets that cannot be covered by the navigation.

26. Graphical user interface according to any one of the preceding claims 22 to 25, wherein a group of dissimilar technologies (91, 92, 93) is depicted in the decision space (D), which correspond to different radiotherapy machines in the machine room (M), in particular according to the type of radiation they emit.

## Revendications

1. **Procédé permettant de rechercher et de définir** un plan thérapeutique parmi une pluralité de propositions de plans pour le traitement du cancer, chaque proposition de plan représentant, en tant que solution, un point dans un espace de navigation (N) qui contient une pluralité de paramètres de réglage et qui définit, dans un espace machine (M), un réglage pour les doses de rayonnement à administrer par un appareil de radiothérapie destiné au traitement du cancer ;
(a) l'espace de navigation (N) contient au moins un front de Pareto (200), derrière ou sur lequel se trouvent des plans de traitement disponibles en tant que solutions possibles et parmi lesquels un utilisateur navigue, une première pluralité de solutions disponibles étant conservée dans une mémoire, lesquelles ont été calculées au préalable en tant que propositions de plans et sont récupérées ou lues à partir de la mémoire pour être affichées ;
(b) les propositions de plans extraites ou lues sont affichées sur un écran et utilisées par l'utilisateur pour la navigation , parmi lesquelles se trouve un grand nombre de solutions pareto-optimales avec des valeurs physiques ou des évaluations de doses, mais sont également affichées des solutions quasi-pareto-optimales, situées dans une plage de tolérance ne dépassant pas ±5 % par rapport à l'optimum de Pareto ;
(c) ce qui permet de créer un premier ensemble important de points comprenant les valeurs physiques ou les évaluations des doses, de les enregistrer en mémoire et d'en calculer un deuxième ensemble de points définissant des objectifs cliniques ;
(d) un espace de décision (D) étant prévu et représenté, dans lequel au moins les objectifs cliniques sont reportés sur des axes d'objectifs (76, 876, 776) ;
sans possibilité de naviguer dans l'espace de décision (D) sur les axes cibles des objectifs cliniques, mais en sélectionnant de manière binaire, à la manière de restricteurs dans l'espace de décision, quelles propositions de plan ne seront pas utilisées comme plans thérapeutiques, et en identifiant et en définissant ainsi un plan thérapeutique souhaité parmi la multitude de propositions de plan.

2. Procédé selon la revendication 1, dans lequel sont également représentés dans l'espace de décision (D) les axes cibles (166) avec leurs critères (176) qui contiennent des valeurs physiques de doses, en particulier pour un volume d'intérêt ( , Volume of Interest, ) indiquant une valeur moyenne de dose correspondante sous la forme d'une norme mathématique (convexe) de la dose.

3. Procédé selon la revendication 1, dans lequel, parmi les axes cibles représentés dans l'espace de décision (D), figure un axe cible (766) qui indique, en tant qu'objectif passif, une plage de temps pour l'un de plusieurs appareils thérapeutiques, pendant laquelle un traitement sur cet appareil thérapeutique est possible ou avant laquelle un traitement sur cet appareil thérapeutique n'est pas possible.

4. Procédé selon la revendication 1 ou 3, dans lequel, parmi les axes cibles représentés dans l'espace de décision (D), figure un axe cible (666) qui représente une combinaison logique d'objectifs issus de l'espace de navigation (N), en particulier une condition selon laquelle
au moins l'une des deux glandes salivaires doit survivre à la radiothérapie identifiée et définie ;
ou
au moins un appareil visuel parmi deux appareils visuels doit survivre à la radiothérapie identifiée et définie, un appareil visuel étant constitué d'un nerf optique et d'un œil ;
ou
au moins un organe parmi les organes présents en double, tels que les poumons, doit survivre à la radiothérapie identifiée et définie.

5. Procédé selon l'une des revendications précédentes, dans lequel la plage de tolérance autour de l'optimum de Pareto est déterminée par une imprécision de calcul, une imprécision de position, une erreur de modélisation ou une imprécision numérique.

6. Procédé selon l'une des revendications précédentes, dans lequel, sur au moins l'un des axes cibles, une zone entière est exclue de manière contiguë des cibles cliniques dans l'espace de décision (D), à la suite de la définition d'un restricteur (100, 101) associé à cet axe cible.

7. Procédé selon la revendication 6, dans lequel une autre zone est exclue de manière contiguë, à la suite de la mise en place d'un autre restricteur (100, 101) appartenant à un autre axe cible dans l'espace de décision (D), ce qui permet de filtrer de préférence différentes zones sur différents axes cibles dans l'espace de décision (D).

8. Procédé selon l'une des revendications précédentes, dans lequel plusieurs axes cibles sont regroupés dans l'espace de décision (PR1, PR2, PR3).

9. Procédé selon la revendication 8, dans lequel le regroupement comprend des cibles nécessaires, des cibles importantes et des cibles non détectables par la navigation dans l'espace de navigation.

10. Procédé selon l'une des revendications précédentes, dans lequel un groupe de technologies différentes (91, 92, 93) est représenté dans l'espace de décision (D).

11. Procédé selon la revendication 10, dans lequel une fonction de notation attribue automatiquement des marqueurs (56, 156) à certaines propositions de plan, en particulier à une seule proposition de plan, de préférence la meilleure possible, pour une technologie donnée.

12. Procédé selon la revendication 11, dans lequel le marqueur (56, 156) est placé sur un curseur (66, 166) qui comporte un limiteur.

13. Procédé selon l'une des revendications précédentes, dans lequel une recombinaison des objectifs représentés dans l'espace de décision (D) pour revenir dans l'espace de navigation (N) n'est pas possible d'un point de vue mathématique.

14. Procédé selon l'une des revendications précédentes, dans lequel le premier ensemble de points comporte des évaluations physiques ou des valeurs de dose supérieures à 1 000.

15. Procédé selon l'une des revendications précédentes, dans lequel le deuxième ensemble de points calculé, qui représente respectivement des cibles cliniques, est converti dans l'espace de décision (D), tandis que seuls les points qui sont pareto-optimaux dans l'espace de décision (D) sont représentés.

16. Procédé selon l'une des revendications précédentes, dans lequel des points issus du premier ensemble de points, correspondant aux valeurs physiques ou aux évaluations de doses, sont reportés sur des curseurs (66, 166) dans l'espace de décision (D), chaque curseur comportant un limiteur (106, 105).

17. Procédé selon l'une des revendications précédentes, dans lequel le premier ensemble de points comportant des valeurs physiques ou des évaluations de valeurs de dose est élargi par des interpolations entre les propositions de planification consultées ou lues lors de la navigation de l'utilisateur, ce premier ensemble de points élargi est stocké en mémoire et, à partir de celui-ci, est calculé le deuxième ensemble de points qui définit des objectifs cliniques.

18. Procédé selon l'une des revendications précédentes, dans lequel le calcul du deuxième ensemble de points ne présente pas de relation 1:1 avec le premier ensemble de points.

19. Procédé selon l'une des revendications précédentes, dans lequel le premier ensemble de points, comprenant des valeurs physiques ou des évaluations de valeurs de dose, contient des grandeurs cibles qui représentent une limite inférieure de dose, une limite supérieure de dose ou une valeur moyenne de dose.

20. Procédé selon l'une des revendications précédentes, dans lequel le premier ensemble de points est calculé selon des fonctions convexes pendant la navigation dans l'espace de navigation (N).

21. Procédé selon l'une des revendications précédentes, dans lequel le premier ensemble de points comprenant des valeurs physiques ou des évaluations de valeurs de dose contient des valeurs moyennes de dose, celles-ci étant calculées différemment selon l'organe : selon la norme maximale pour les organes en série, et selon la moyenne arithmétique pour les organes en parallèle.

22. **Interface utilisateur graphique techniquement utilisable** pour trouver un plan thérapeutique parmi des propositions de plans pour le traitement du cancer, chaque solution, en tant que proposition de plan, représentant un point dans un espace de navigation (N) et formant une solution de Pareto qui contient une multitude de paramètres de réglage permettant de définir, dans un espace machine (M), les réglages des doses de rayonnement à administrer par un appareil de radiothérapie destiné au traitement du cancer ;
(a) comprenant un espace de navigation (N) qui présente au moins un front de Pareto (200), une pluralité de propositions de plans disponibles étant conservées dans une mémoire, lesquelles ont été calculées au préalable et sont récupérées ou lues à partir de la mémoire en vue de leur représentation ; les solutions récupérées ou lues étant affichées sur un écran et pouvant être utilisées par l'utilisateur à l'aide de curseurs pour sa navigation ;
(c) un autre espace de décision (D), représenté séparément, étant prévu, dans lequel les points navigables de l'espace de navigation sont représentés sur des axes cibles (66, 166, 266), sans possibilité de naviguer dans l'espace de décision (D), mais en sélectionnant de manière binaire, à la manière d'un restricteur, sur les axes cibles de l'espace de décision à l'aide de curseurs, les propositions de plans qui ne sont pas utilisables ou ne doivent pas être utilisées comme plans thérapeutiques ;
(d) Dans l'espace de décision (D), sont inclus et représentés au moins les axes cibles (766) destinés au filtrage qui ne sont pas représentés dans l'espace de navigation (N) à des fins de navigation.

23. Interface utilisateur graphique selon la revendication 22, dans laquelle les axes cibles de l'espace de navigation (N) sur lesquels une navigation quasi-continue est possible sont également représentés dans l'espace de décision (D), notamment sous forme de curseurs (766, 666).

24. Interface utilisateur graphique selon l'une des revendications précédentes 22 ou 23, dans laquelle les axes cibles sont représentés par groupes (PR1, PR2, PR3) dans l'espace de décision.

25. Interface utilisateur graphique selon la revendication 22, dans laquelle le regroupement comprend des cibles nécessaires, des cibles importantes et des cibles non accessibles par la navigation.

26. Interface utilisateur graphique selon l'une des revendications précédentes 22 à 25, dans laquelle un groupe de technologies différentes (91, 92, 93) est représenté dans l'espace de décision (D), lesquelles correspondent à différentes machines de radiothérapie dans la salle des machines (M), notamment en fonction du type de rayonnement qu'elles émettent.
